# EUROPÄISCHE PATENTSCHRIFT

(11) **EP 0 911 005 B1**
(45) Veröffentlichungstag und Bekanntmachung des Hinweises auf die Patenterteilung: **05.03.2003**
(21) Anmeldenummer: 98118612.5
(22) Anmeldetag: 01.10.1998
(51) Int. Cl.: A61F 5/37, A61F 13/10

(54) **Medizinische Bandage**
Medical bandage
Bandage médical

(30) Priorität: 16.10.1997 DE 19745705
(43) Veröffentlichungstag der Anmeldung: 28.04.1999
(73) Patentinhaber: BSN medical GmbH & Co. KG, 20253 Hamburg (DE)
(72) Erfinder: Herzberg, Thorsten, 21149 Hamburg (DE); Stradt, Thorsten, 22453 Hamburg (DE); Albrod, Andreas Dr., 21217 Seevetal (DE); Votsch, Harald, 22301 Hamburg (DE); Reichert, Oliver, 22761 Hamburg (DE); Andrews, Arthur-Hugh, 25337 Kölln-Reisiek (DE); Rosenbaum, Brigitte, 21079 Hamburg (DE)
(74) Vertreter: Sherrard-Smith, Hugh

(56) Entgegenhaltungen:
- EP-A- 0 198 482
- EP-A- 0 589 663
- DE-U- 9 416 590
- US-A- 4 446 858
- US-A- 5 405 312

## Beschreibung

Die Erfindung beschreibt eine medizinische Armbandage mit Einfassung und Führung des Unterarmes und des Oberarms beziehungsweise der Schulter.

Orthopädischen Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine dreidimensionale Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigen Material, zum Beispiel aus Neopren, Gewirke, oder Geweben. Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.
Das Zusammenfügen kann durch Nähen, Kleben oder andere übliche Verfahren erfolgen.

Eine weitere Möglichkeit der Herstellung der medizinischen Bandagen ist das Formstricken mit Flachstrick- oder Rundstrickmaschinen. Dieses Verfahren ist jedoch von der Möglichkeit der Formgebung und der Materialauswahl beschränkt. Insbesondere ist nur eine zweidimensionale Formgebung möglich. Die dritte Dimension ist nur durch nachträgliches Zusammennähen erhältlich.

Es sind auch Bandagen bekannt, bei denen Schaumgummi durch Kompressionsmolding zu unterschiedlichen Dicken verformt wird. Dadurch sollen durch die unterschiedliche Dichte des Schaumstoffs nach der Verformung die elastischen Eigenschaften des Materials lokal verändert werden. Beispielhaft ist eine derartige Bandage in WO 95/32690 beschrieben.

Weiterhin ist bekannt, thermoplastisches Kunststoffplattenmaterial anatomisch formgerecht zu orthopädischen Orthesen und Prothesen zu formen. Diese Materialien, wie beispielsweise Polyethylen (HDPE), Polypropylen oder ein Copolymer PP, besitzen einen thermoplastischen Umformungsbereich von ca. 170 °C bis 250 °C und sind nach dem Erkalten weitestgehend rigide, so daß sie nicht für medizinische Bandagen zum Einsatz kommen.

Aus der DE P 43 14 785 ist ein Bandagensystem insbesondere für akromioklavikulare Luxationen und laterale Claviculafrakturen bekannt. Das Bandagensystem setzt sich zusammen aus einem Schlauchteil, das den Unter- und den Oberarm aufnimmt und das aus einem radialelastischen, in Längsrichtung jedoch im wesentlichen undehnbaren Gewebematerial besteht, einem Trageband, einem Halteband, Verschlußorganen, die an den genannten Bändern zur Bildung von Halteschlaufen vorgesehen sind, sowie einem Zuggurt. Eine derartige Bandage ist zwar geeignet, ein Abklingen der dargelegten Indikationen zu erreichen, ist aber sehr aufwendig herzustellen. Weiterhin ist es durch die schlauchförmige Konstruktion oftmals schwierig für den Patienten, die Bandage schmerzfrei anzulegen.

Die allgemein bekannten Bandagen für diesen Indikationsbereich weisen eine sehr geschlossene Konstruktion auf, die zwangsläufig dazu führt, daß die Bandagen nur unter Hilfe einer zusätzlichen Person schmerzfrei angelegt werden können. Sehr oft führen diese Bandagen auch zu einem unangenehmen Schwitzen mit Wärmestau aufgrund der meist geschlossen Konstruktion.

Die einfacher konstruierten Bandagen, die zumeist nur aus zwei Gurten bestehen, haben nicht die Problematik des Schwitzens, sind aber von ihrer Konstruktion in Bezug auf ihrer Therapiesicherheit und Funktion meist zweifelhaft und somit ungenügend.

Aufgabe der Erfindung war es daher, eine Bandage zur Verfügung zu stellen, die eine offene Konstruktion und eine hohe Therapiesicherheit aufweist und die schmerzfrei ohne Hilfe von seiten Dritter angelegt werden kann.

Gelöst wird diese Aufgabe durch eine Bandage, wie sie in Anspruch 1 dargelegt ist. Gegenstand der Unteransprüche sind dabei vorteilhafte Weiterbildungen der Bandage.

Demgemäß besteht die Bandage für den Schulter- und Oberarmbereich aus einem Oberarmteil und einem Unterarmteil. Das Oberarmteil ist U-förmig ausgebildet ist, wobei der Mund, der den Bereich des Oberarmteils bezeichnet, in dem der erste und der zweite Schenkel zusammenlaufen, auf der Schulter des Patienten aufliegt, der erste Schenkel sich anterior bis annähernd zum Ellenbogengelenk erstreckt und der zweite Schenkel sich posterior bis annähernd zum Ellenbogengelenk erstreckt.
Das Unterarmteil ist aus drei Abschnitten gebildet, einem ersten Abschnitt, der bei in einem Winkel von ungefähr 90° anterior angewinkeltem Unterarm im ulnaren Unterarmbereich liegt, einem zweiten Abschnitt, der mit dem zweiten Schenkel des Oberarmteils verbunden ist und der anterior über das Ellenbogengelenk verläuft und in dem ersten Abschnitt mündet, einem dritten Abschnitt, der mit dem ersten Schenkel des Oberarmteils verbunden ist und posterior über das Ellenbogengelenk verläuft und in dem ersten Abschnitt mündet, wobei die Enden des zweiten und des dritten Abschnitts miteinander verbunden sind. Weiterhin sind an dem Oberarmteil ein Tragegurt und an dem Unterarmteil ein Haltegurt angebracht sind, um die erfindungsgemäße Bandage am Körper zu fixieren.
Bei unvernähter Bandage schließen der zweite Abschnitt und der dritte Abschnitt des Unterarmteils einen Winkel von 20° bis 150° ein, bevorzugt 60° bis 120°.

Das Oberarmteil und das Unterarmteil können mittels einer Klettverbindung, mittels Druckknöpfen oder Nähten miteinander verbunden sein. Insbesondere ist eine verstellbare Verbindung vorhanden, um den individuellen Längenverhältnissen der Extremitäten gerecht zu werden und so die Paßformeffizienz zu verbessern.

In dem Oberarmteil sind in einer bevorzugten Ausführungsform an den Enden der Schenkel elastische Abschnitte vorhanden.

Diese elastischen Abschnitte im distalen Oberarmteil sorgen für eine ideale individuelle Anpassung an die anatomische Formgebung im Bereich der Schulter und Oberarms sowie des Ellenbogens.
Dadurch werden die Schulterpartie und insbesondere der Oberarmkopf sicher geführt.

Als vorteilhaft hat sich erwiesen, wenn die Bestandteile der Bandage aus einem textilkaschierten Schaumstoff oder Vliesstoff bestehen oder aus einem geformten Schaumstorf oder Vliesstoff.

In einer weiteren vorteilhaften Ausführungsform ist in dem Oberarmteil zentral im Mundbereich eine entsprechend geformte Verstärkung vorhanden. Diese Verstärkung kann ebenfalls U-förmig ausgebildet sein. Die Verstärkung besteht vorzugsweise aus Kunststoff und weist die Form eines Stabes oder einer Schiene auf.

In einer weiteren vorteilhaften Ausführungsform weist das Unterarmteil eine Handführung oder Handfixierung auf. Die Handführung kann optional, entsprechend der Therapie und der Indikation, individuell angepaßt werden, um somit einen optimalen Sitz sicherzustellen.
Die Handführung führt die Hand und verhindert so eine übermäßige Ulnarflexion, des weiteren wirkt diese einer Überdehnung der Sehnen und des Bandapparates entgegen, die meist im volaren Bereich sehr empfindlich sind.

Die Handführung kann aus zwei Zügeln bestehen, die sich am ersten Abschnitt des Unterarmteils anschließen, und zwar im distalen Unterarmbereich. Die beiden Zügel schließen einen Winkel von 10° bis 160° ein, insbesondere 90°. Mittels am distalen Ende der Zügel vorhandene Klettverschlüsse können die Zügel miteinander verbunden werden, und somit wird die Hand sicher fixiert.

Auch beim Unterarmteil hat sich als vorteilhaft erwiesen, wenn Verstärkungen im ersten Abschnitt eingearbeitet sind, die die Form eines Stabes oder einer Schiene aufweisen und die aus Kunststoff hergestellt werden.

Das Oberarmteil kann mit einem Tragegurt, der von der Schulter zum anterialen Thoraxbereich verläuft, nach ventral zum Unterarm im Bereich des Handgelenkes fixiert werden, wobei der Tragegurt im Bereich des Überganges der Halswirbelsäule zum Schultergürtel ein partielles Polster beinhalten kann.
Der Tragegurt sorgt für eine sichere Positionierung des Oberarmteils und führt zu einer Gewichtsentlastung des Unterarmes, was sich positiv auf das Schultergelenk auswirkt. Üblicherweise unterliegt das Schultergelenk einer ständigen Krafteinwirkung aufgrund des Gewichts des Unterarms. Diese Zugkraft wirkt sich nachteilig auf den Sehnen- und Bandapparat des Schultergelenkes aus.

Der Haltegurt vom Unterarmteil verläuft vorteilhafterweise von der Handregion kommend nach dorsal im Lendenbereich zum distalen Oberarm und umschließt diesen von posterior nach anterior verlaufend nach lateral.
Der Haltegurt sorgt für eine sichere Fixierung des Unterarmes am ventralen Leib. Durch die Fassung des distalen Oberarmes wird die Bewegung im Sinne der Flexion beziehungsweise Extension sowie der Antetorsion des Schultergelenkes kontrolliert geführt.

Die Fixierung der Gurte kann durch beispielsweise durch Klettverschlüsse oder durch Druckknöpfe erfolgen.

Vorzugsweise bestehen die Gurte aus einem laminierten Schaumstoff oder einem laminierten Vliesstoff.

Schließlich hat es sich als vorteilhaft erwiesen, wenn die Gurte einen hohen Polstereffekt und unter Belastung von ungefähr 50 N eine bevorzugte Längsdehnung von kleiner 35%, besonders bevorzugt eine Längsdehnung von kleiner 10 %, aufweisen.

Die erfindungsgemäße Bandage dient insbesondere als eine posttraumatische und postoperative Spezialbandage für Verletzungen im Schulter- und Oberarmbereich, zum Beispiel Distorsionen, Rotatorenmanschettenverletzungen und sonstigen Verletzungen, operativen Eingriffen und chronischen Erkrankungen, die eine Ruhigstellung der Schulter erfordern.

Eine solche Spezialbandage muß die erfindungsgemäßen Eigenschaften aufweisen, um indikationsgerecht wirken zu können:
- eine explizite Paßform, um eine sichere und stabile Fixation der Schulter und des Oberarmes zu gewährleisten.
- ein gut eingefaßtes Schultergelenk mit entsprechender Fixationsübergang zum thorakalen Bereich

Je nach Indikationsfeld ist ebenso eine Unterarmfixierung mit integrierter Handführung erforderlich, um eine hohe Sicherheit des Therapieerfolges zu erreichen. Ähnlich wie bei üblicher Gipstherapie, werden bei einem betroffenen Gelenk immer ein oder zwei Gelenke überbrückt, um eine wirkliche Therapie zu sichern.

Diese Kriterien werden erfüllt durch die erfindungsgemäße Ausgestaltung des Oberarm- und des Unterarmteils, die den anatomischen Gegebenheiten des Armes und der Schulter entsprechen.

Die offene Konstruktion ermöglicht darüber hinaus ein einfaches und schmerzfreies Anlegen der erfindungsgemäßen Bandage. Dies ist ein besonderer Vorteil, zumal der Patient sich die Bandage selbst anlegen kann. Darüber hinaus ist ein als sehr unangenehm empfundenes Schwitzen des Patienten sehr stark unterbunden.

Im folgenden soll eine besonders vorteilhafte Ausführung der erfindungsgemäßen Bandage mittels mehrerer Figuren beschreiben werden, ohne damit die Erfindung unnötig einschränken zu wollen.

Im einzelnen zeigen
- die Figur 1: das Oberarmteil,
- die Figur 2: das Unterarmteil mit aufgetrennten Nähnähten und
- die Figur 3: die am rechten Arm des Patienten angelegte erfindungsgemäße Bandage, bestehend aus Oberarm- und Unterarmteil samt Verbindungsvorrichtungen und Gurten.

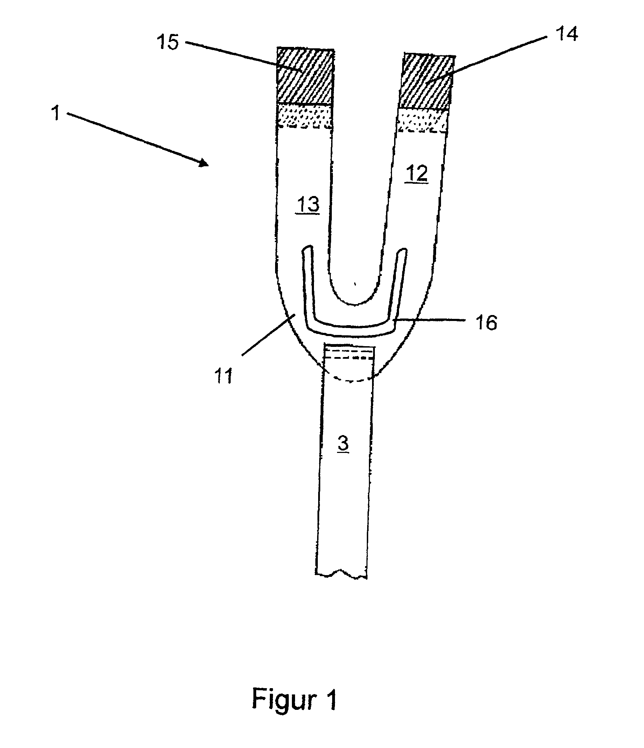

In der Figur 1 ist das Oberarmteil 1 dargestellt. Das Oberarmteil 1 ist dabei U-förmig derartig ausgeformt, daß es sich hervorragend an die anatomischen Gegebenheiten des Oberarms und der Schulter des Patienten anpaßt. Das Oberarmteil 1 besteht aus zwei Schenkeln 12, 13, die im Mund 11 zusammenlaufen. Der Mund 11 befindet sich bei angelegter Bandage 100 auf der Schulter des Patienten. Weiterhin ist im Mund 11 eine Verstärkung 16 eingearbeitet, die stabförmig ausgebildet ist und aus Kunststoff besteht. Zentral am Mund 11, und zwar gegenüber den Schenkeln 12, 13 ist der Tragegurt 3 angenäht.
An den Enden der im wesentlichen rechteckig geformten Schenkel 12, 13 befinden sich zwei elastische Abschnitte 14, 15, die aufgrund ihrer Flexibilität einen optimalen Sitz der Bandage 100 gewährleisten. Die elastischen Abschnitte 14, 15 werden mit dem zweiten 22 beziehungsweise dritten Abschnitt 23 des Unterarmteils 2 vernäht.

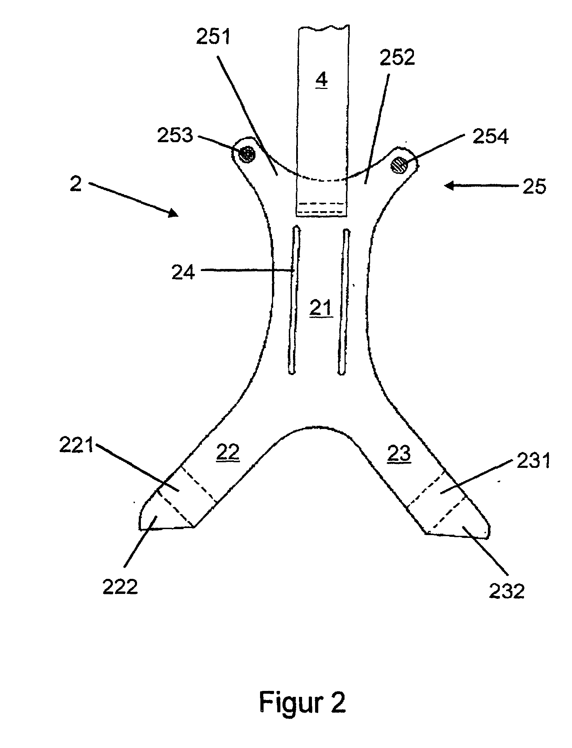

In der Figur 2 ist das Unterarmteil 2 gezeigt, wobei die vorhandenen Nähte aufgetrennt sind. Das Unterarmteil 2 weist insgesamt drei Abschnitte 21, 22, 23 auf. Der zentrale erste Abschnitt 21 liegt dabei im ulnaren Unterarmbereich. An den ersten Abschnitt 21 schließen sich der zweite Abschnitt 22 an sowie der dritte Abschnitt 23, die einen Winkel von ungefähr 90° einschließen. Die Enden 222, 232 der Abschnitte 22, 23 sind bei der fertigen Bandage 100 miteinander vernäht. Auf diese Weise entsteht eine schalenförmige Ausbuchtung, die an die anatomischen Gegebenheiten des Ellenbogengelenks hervorragend angepaßt ist und in die der Ellenbogen aufgenommen werden kann.
Im Bereich 221 ist der elastische Abschnitt 14 des ersten Schenkels 12 vom Oberarmteil angenäht, im Bereich 231 der elastische Abschnitt 15. Der erste Abschnitt 21 trägt des weiteren schienenförmige Verstärkungen 24, die ebenfalls aus Kunststoff gefertigt sind. In der hier dargestellten vorteilhaften Ausführungsform der Bandage 100 ist am Unterarmteil 2 eine Handfixierung 25 angeformt, die sich aus zwei Zügel 251, 252 zusammensetzt, die einen Winkel von 90° einschließen. An den Enden der Zügel 251, 252 befinden sich zwei Klettverschlüsse 253, 254, die miteinander verbunden werden, so daß die Handfixierung 25 die Hand des Patienten umschließt. Zentral an der Handfixierung 25 ist der Haltegurt 4 befestigt.

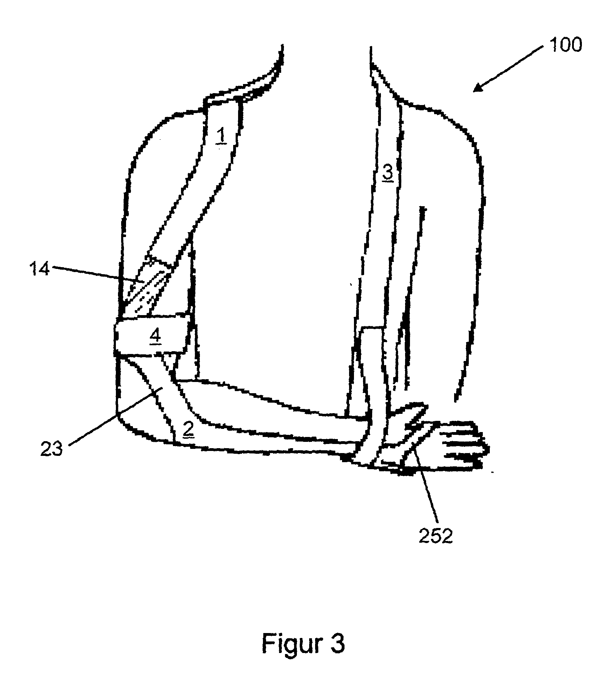

Die in Figur 3 gezeigte Bandage 100, die an der rechten Schulter beziehungsweise dem rechten Unterarm des Patienten angelegt ist, setzt sich im wesentlichen aus dem Oberarmteil 1 und dem Unterarmteil 2 zusammen.

Das Oberarmteil 1 wird mit einem Tragegurt 3, der von der Schulter zum Nackenbereich verläuft, nach ventral zum Unterarm im Bereich des Handgelenkes fixiert. Die Fixierung des Tragegurts 3 erfolgt mittels eines Klettverschlusses, so daß um den Unterarm eine Schlaufe gebildet ist.
Das Unterarmteil 2 wird mit einem Haltegurt 4 fixiert, der von der Handregion kommend nach dorsal im Lendenbereich zum distalen Oberarm verläuft. Die Fixierung des Haltegurts 4 erfolgt ebenfalls durch einen Klettverschluß.
Das gesamte Gurtführungssystem sorgt so in einer Symbiose für die Sicherung der Position der Bandage am Patienten.

## Patentansprüche

1. Bandage 100 für den Schulter- und Oberarmbereich, bestehend aus einem Oberarmteil 1 und einem Unterarmteil 2, wobei
das Oberarmteil 1 U-förmig ausgebildet ist, wobei der in dem Bereich des Oberarmteils 1, in dem der erste 12 und der zweite 13 Schenkel zusammenlaufen, liegende Mund 11 auf der Schulter aufliegt, der erste Schenkel 12 sich anterior bis annähernd zum Ellenbogengelenk erstreckt und der zweite Schenkel 13 sich posterior bis annähemd zum Ellenbogengelenk erstreckt, und
das Unterarmteil 2 aus drei Abschnitten 21, 22, 23 gebildet ist,
einem ersten Abschnitt 21, der bei in einem Winkel von ungefähr 90° anterior angewinkeltem Unterarm im ulnaren Unterarmbereich liegt,
einem zweiten Abschnitt 22, der mit dem zweiten Schenkel 13 des Oberarmteils 1 verbunden ist und der anterior über das Ellenbogengelenk verläuft und in den ersten Abschnitt 21 mündet,
einem dritten Abschnitt 23, der mit dem ersten Schenkel 12 des Oberarmteils 1 verbunden ist und posterior über das Ellenbogengelenk verläuft und in den ersten Abschnitt 21 mündet, wobei die Enden des zweiten 22 und des dritten Abschnitts 23 miteinander verbunden sind, wobei an dem Oberarmteil 1 ein Tragegurt 3 und an dem Unterarmteil 2 ein Haltegurt 4 angebracht sind.

2. Bandage nach Anspruch 1, **dadurch gekennzeichnet, daß** die Bestandteile aus einem textilkaschierten Schaumstoff oder Vliesstoff bestehen.

3. Bandage nach Anspruch 1, **dadurch gekennzeichnet, daß** die Bestandteile aus einem geformten Schaumstoff oder Vliesstoff bestehen.

4. Bandage nach Anspruch 1, **dadurch gekennzeichnet, daß** in dem Oberarmteil 1 an den Enden der Schenkel 12, 13 elastische Abschnitte 14, 15 vorhanden sind.

5. Bandage nach Anspruch 1, **dadurch gekennzeichnet, daß** in dem Oberarmteil 1 zentral im Mundbereich 11 eine entsprechend geformte Verstärkung 16 vorhanden ist.

6. Bandage nach Anspruch 1, **dadurch gekennzeichnet, daß** das Unterarmteil 2 eine Handführung oder Handfixierung 25 aufweist.

7. Bandage nach Anspruch 1, **dadurch gekennzeichnet, daß** im ersten Abschnitt 21 des Unterarmteils Verstärkungen 24 eingearbeitet sind.

8. Bandage nach Anspruch 1, **dadurch gekennzeichnet, daß** das Oberarmteil 1 mit einem Tragegurt 3, der von der Schulter zum anterialen Thoraxbereich verläuft, nach ventral zum Unterarm im Bereich des Handgelenkes fixiert wird.

9. Bandage nach Anspruch 1, **dadurch gekennzeichnet, daß** der Tragegurt 3 im Bereich des Überganges der Halswirbelsäule zum Schultergürtel ein partielles Polster beinhaltet.

10. Bandage nach Anspruch 1, **dadurch gekennzeichnet, daß** der Haltegurt 4 vom Unterarmteil 2 von der Handregion kommend nach dorsal im Lendenbereich zum distalen Oberarm verläuft und diesen von posterior nach anterior verlaufend nach lateral umschließt.

11. Bandage nach Anspruch 1, **dadurch gekennzeichnet, daß** die Gurte 3, 4 aus einem laminierten Schaumstoff oder einem laminierten Vliesstoff bestehen.

12. Bandage nach Anspruch 1, **dadurch gekennzeichnet, daß** die Gurte 3, 4 einen hohen Polstereffekt und unter Belastung von annähernd 50 N eine bevorzugte Längsdehnung von kleiner 35%, besonders bevorzugt eine Längsdehnung von kleiner 10 %, aufweisen.

## Claims

1. Bandage 100 for the shoulder and upper arm region, consisting of an upper arm part 1 and a forearm part 2, wherein
the upper arm part 1 is U-shaped, with the mouth 11 in the region of the upper arm part 1 in which the first and second legs 12 and 13 respectively of the "U" meet, resting on the shoulder, the first leg 12 of the "U" extending approximately to the elbow joint in front of the patient's arm, and the second leg 13 of the "U" extending approximately to the elbow joint behind the patient's arm, and
the forearm part 2 is formed from three portions 21, 22, 23,
a first portion 21 which is located in the ulnar region of the forearm when the forearm is bent in front of the patient at an angle of approximately 90°,
a second portion 22 which is joined to the second leg 13 of the upper arm part 1 and extends beyond the elbow joint in front of the patient's arm and merges with the first portion 21, and
a third portion 23 which is joined to the first leg 12 of the upper arm part 1 and extends beyond the elbow joint behind the patient's arm and merges with the first portion 21, the ends of the second and third portions 22 and 23 respectively being joined to each other, a suspension strap 3 being attached to the upper arm part 1 and a restraining strap 4 being attached to the forearm part 2.

2. Bandage according to Claim 1, **characterized in that** the component parts are made from a textile-laminated foam material or non-woven fabric.

3. Bandage according to Claim 1, **characterized in that** the component parts are made from a moulded foam material or non-woven fabric.

4. Bandage according to Claim 1, **characterized in that** elastic portions 14, 15 are provided at the ends of the legs 12, 13 in the upper arm part 1.

5. Bandage according to Claim 1, **characterized in that** a suitably shaped reinforcement 16 is provided centrally in the mouth region 11 in the upper arm part 1.

6. Bandage according to Claim 1, **characterized in that** the forearm part 2 has a hand guide or hand immobilizer 25.

7. Bandage according to Claim 1, **characterized in that** reinforcements 24 are incorporated in the first portion 21 of the forearm part.

8. Bandage according to Claim 1, **characterized in that** the upper arm part 1 is secured with a suspension strap 3 which extends from the shoulder to the front of the chest and continues down to the forearm in the region of the wrist.

9. Bandage according to Claim 1, **characterized in that** the suspension strap 3 contains partial padding in the region of the transition from the cervical vertebral column to the shoulder strap.

10. Bandage according to Claim 1, **characterized in that** the restraining strap 4 of the forearm part 2 extends from the hand region dorsally in the lumbar region to the distal upper arm and surrounds the latter, extending from behind forwards and to the side.

11. Bandage according to Claim 1, **characterized in that** the straps 3, 4 are made of a laminated foam material or laminated non-woven material.

12. Bandage according to Claim 1, **characterized in that** the straps 3, 4 possess a high cushioning effect and have a preferred elongation of less than 35%, and an especially preferred elongation of less than 10%, under a loading of approximately 50 N.

## Revendications

1. Bandage 100 pour la région de l'épaule et de l'arrière-bras, comprenant une partie d'arrière-bras 1 et une partie d'avant-bras 2, la partie d'arrière-bras 1 étant conformée en U, le coeur 11 situé dans la région de la partie d'arrière-bras 1, au niveau de laquelle la première branche 12 se raccorde à la seconde branche 13, reposant sur l'épaule, la première branche 12 s'étendant côté face jusque dans la région de l'articulation du coude et la seconde branche 13 s'étendant côté dos sensiblement jusque dans la région de l'articulation du coude, et
la partie d'avant-bras 2 étant formée de trois tronçons 21, 22, 23, à savoir d'un premier tronçon 21 qui, l'avant-bras étant replié sur le devant sous un angle d'environ 90°, est situé dans la région ulnaire de l'avant-bras,
d'un deuxième tronçon 22, qui est lié à la seconde branche 13 de la partie d'arrière-bras 1 et qui, côté face passe sur l'articulation du coude et se raccorde au premier tronçon 21,
d'un troisième tronçon 23, qui est lié à la première aile 12 de la partie d'arrière-bras 1 et côté dos passe sur l'articulation du coude et se raccorde au premier tronçon 21, les extrémités des deuxième 22 et troisième 23 tronçons étant liées l'une à l'autre, une sangle de support 3 étant fixée à la partie d'arrière-bras 1 et une sangle de maintien 4 étant fixée à la partie d'avant-bras 2.

2. Bandage selon la revendication 1, **caractérisé en ce que** les éléments sont en un matériau expansé doublé de produit textile ou de produit non tissé.

3. Bandage selon la revendication 1, **caractérisé en ce que** les éléments sont en un matériau expansé ou en produit non tissé préformé.

4. Bandage selon la revendication 1, **caractérisé en ce que** dans la partie d'arrière-bras 1, des parties 14, 15 élastiques sont prévues aux extrémités des branches 12, 13.

5. Bandage selon la revendication 1, **caractérisé en ce que** dans la partie d'arrière-bras 1, un renfort 16 de forme adaptée est prévu dans la partie coeur 11.

6. Bandage selon la revendication 1, **caractérisé en ce que** la partie d'avant-bras 2 comporte un guide pour la main ou une attache 25 pour la main.

7. Bandage selon la revendication 1, **caractérisé en ce que** des renforts 24 sont intégrés dans le premier tronçon 21 de la partie d'avant-bras.

8. Bandage selon la revendication 1, **caractérisé en ce que** la partie d'arrière-bras 1 est fixée côté ventral à la partie d'avant-bras, dans la région du poignet, par une sangle de support 3, qui s'étend de l'épaule en direction de la zone antérieure du thorax.

9. Bandage selon la revendication 1, **caractérisé en ce que** la sangle de support 3, dans la région de transition entre la zone cervicale et la sangle d'épaule, comporte un rembourrage local.

10. Bandage selon la revendication 1, **caractérisé en ce que** la sangle de maintien 4, partant de la partie d'avant-bras 2, de la région de la main, s'étend en direction du dos dans la région des reins, jusqu'à l'arrière-bras distal et l'entoure latéralement, de l'arrière vers l'avant.

11. Bandage selon la revendication 1, **caractérisé en ce que** les sangles 3 et 4 sont formées d'un produit expansé stratifié ou d'un produit non tissé stratifié.

12. Bandage selon la revendication 1, **caractérisé en ce que** les sangles 3 et 4 présentent un effet amortissant élevé et, sous une charge d'environ 50 N présentent un allongement longitudinal préféré inférieur à 35%, plus particulièrement un allongement longitudinal inférieur à 10%.
